# EUROPEAN PATENT SPECIFICATION

(11) **EP 1 703 913 B1**
(45) Date of publication and mention of the grant of the patent: **01.08.2012**
(21) Application number: 05711532.1
(22) Date of filing: 14.01.2005
(51) Int. Cl.: A61K 31/70, C07H 3/02, A61P 39/00

(54) **USE OF RIBOSE IN RECOVERY FROM ANAESTHESIA**
VERWENDUNG VON RIBOSE BEI DER ERHOLUNG NACH EINER NARKOSE
UTILISATION DE RIBOSE POUR FACILITER LA RECUPERATION SUITE A UNE ANESTHESIE

(30) Priority: 14.01.2004 US 536460 P
(43) Date of publication of application: 27.09.2006
(73) Proprietor: RiboCor, Inc., Minneapolis, MN 55428 (US)
(72) Inventor: ST. CYR, John, A., Coon Rapids, MN 55449 (US); PERKOWSKI, David, A., San Clemente, CA 92673 (US)
(74) Representative: Lucas, Brian Ronald
(86) International application number: PCT/US2005/001435
(87) International publication number: WO 2005/067548

(56) References cited:
- US-B1- 6 218 366
- US-B2- 6 511 964
- US-B2- 6 548 483

## Description

### BACKGROUND OF THE INVENTION

It is well known that the pentose sugar ribose is important in the energy cycle as a constituent of adenosine triphosphate (ATP) and nucleic acids. It is also well known that ribose is found only at low concentrations in the diet, and that further, the metabolic process by which the body produces ribose, the pentose phosphate pathway, is rate limited in many tissues.

Ribose is known to improve recovery of healthy dog hearts subjected to global ischemia at normal body temperatures, when administered for five days following removal of the cross clamp. These inventors have previously discovered (United States Patent Number 6, 159, 942) that the administration of ribose enhances energy in subjects who have not been subjected to ischemic insult. In the case of human patients, by the time cardiac surgery is necessary, the condition of the heart and, possibly, the general state of health, are both impaired. Morbidity and mortality following myocardial ischemia which provides a dry working field can increase due to tissue damage. In addition, the patient is under anesthesia for a considerable period of time.

Most anaesthetic techniques act by inducing a reversible disturbance of the central nervous system (CNS). Spinal or epidural application of local anaesthetics produce a localized inhibition of impulse transmission at spinal cord level leading to central nervous blockade where the essential features are segmental loss of sensory and motor function. General anaesthetics administered intravenously act through binding to specific receptors such as opioid or GABA (γ-aminobutyric acid) receptors; however, the mechanisms of action for inhaled anaesthetics are less well described. Regardless of whether the anaesthetic is local or general, depression of CNS function is intended as part of the anesthesia. All bodily processes are slowed down by the CNS depression. In addition, it is usually necessary during extensive surgery to intubate the patient for respiratory support due to paralysis caused by administration of a curare-type drug. In spite of the respiratory support, pulmonary function is less than optimum. The reduced muscle tone of the diaphragm and intercostal muscles leads to atelectasis, with resulting hypoxemia. The reduced or absent muscle tonus of the skeletal muscles may also lead to reduced circulation and localized hypoxia. Likewise, other organ functions such as the kidney and liver function are somewhat suppressed, leading to accumulation of toxic metabolites. In the worse case scenario, brain dysfunction may be irreversible and manifested by subtle loss of cognitive ability, stroke or irreversible coma or cerebral death.

Upon recovery from anesthesia, the patient usually experiences mental and physical compromise for a period of time. For the first month post anesthesia, it is common for the patient to require more sleep, be less alert when awake and have diminished physical strength. Recurring pain from surgery may necessitate the administration of powerful analgesics which can worsen the already compromised mental and physical state.

It would be beneficial to patients undergoing surgery or any intervention requiring general anesthesia to have less impairment of function following anesthesia and a quicker recovery to normal alertness, ambulatory function and strength.

US-A-6218366 (St. Cyr) discloses that the administration of ribose raises the hypoxic threshold of mammals experiencing a hypoxic condition. The presence of an effective amount of ribose in the tissue of a mammal increases the tolerance to hypoxia and decreases the symtoms of hypoxia in mammals experiencing chronic hypoxia due to cardiovascular disease or peripheral vascular disease. Mammals experiency transient hypoxia from high altitude, anaesthesia or exercice are also benefited. In an example ribose is used for pre-operative conditioning of patients. It is suggested that raising the hypoxic threshold any length of time, even five minutes preoperative, with reduce or eliminate those effects of hypoxia such as free radical formation that are not due to depressed energy levels. Then free-radicals and other metabolites that accumulate during hypoxia are throught to be a possible cause of reperfusion injury which delays recovery of the patient. It is further suggested that giving ribose will give better surgical outcome, faster recovery time and maintenance or improvement of myocardial function together with a decreased need for pharmaceutical or additional support following surgery.

### SUMMARY OF THE INTENTION

The invention provides D-ribose for administration to patients undergoing general anaesthesia for use on achieving quicker recovery to normal alertness.>

D-Ribose is administered as a single agent or more preferably in combination with D-Glucose to a patient scheduled for a procedure requiring general anaesthesia. The agent or agents are administered before and after the general anaesthesia. Preferably, the agent or agents are administered before, during and after the general anaesthesia. Most preferably, the agent or agents are administered for one to seven days before surgery, during surgery and for one to seven days following surgery. The agent or agents are administered orally to a patient able to ingest a solution and intravenously during periods when intravenous fluids are administered.

A method of preparation of substantially pure, pyrogen-free ribose suitable for intravenous administration is disclosed. The intravenous dosage given of each agent or agents is from 30 to 300 mg/kg/how, delivered from a solution of from 5 to 30% w/v of pyrogen-free D-Ribose in water. When D-Glucose is to be co-administered, it may be delivered from a solution of from 5 to 30% w/v of D-Glucose in water. The agent or agents to be administered are tapped into an intravenous line and the flow set to delivered from 30 to 300 mg/kg/how agent or agents. Most preferably, pyrogen-free D-Ribose is administered with D-Glucose, each being delivered intravenously at a rate of 100 mg/kg/hour. When the agent or agents are administered orally, from one to 20 grams of D-Ribose is mixed in 200 ml of water and ingested one to four times per day. Most preferably, five grams of D-Ribose and five grams of D-Glucose are dissolved in water and ingested four times per day.

Patients in the intensive care unit (ICU) are administered pyrogen-free D-Ribose as a single agent or more preferably in combination with D-Glucose. The agent or agents are administered intravenously during the stay in the ICU. The intravenous dosage to be given of each agent or agents is from 30 to 300 mg/kg/hour, delivered from a solution of from 5 to 30% w/v of pyrogen-free D-Ribose in water. When D-Glucose is to be co-administrered, it may be delivered from a solution of from 5 to 30% w/v of D-Glucose in water. The agent or agents to be administered are tapped into an intravenous line and the flow set to delivered from 30 to 300 mg/kg/hour agent or agents. Most preferably, pyrogen-free D-Ribose is administered with D-Glucose, each being delivered at a rate of 100 mg/kg/hour. When patients are released from the ICU, it is beneficial to continue the administration of the agent or agents. Intravenous administration will be continued while an IV line is in place. When the agent or agents are administered orally, from one to 20 grams of D-Ribose is mixed in 200 ml of water and ingested one to four times per day. Most preferably, five grams of D-Ribose and five grams of D-Glucose are dissolved in water and ingested four times per day.

### DETAILED DESCRIPTION OF THE INVENTION

The following examples are given to show how the invention has been or is to be practiced. In particular, it will be noted that in most of the examples, it is suggested that D-Glucose be given along with D-Ribose. It should be noted that the administration of D-Glucose is advised not as a therapy, but to avoid the hypogylcemia that can occur when D-Ribose is given. If it has been determined that a particular subject does not show hypoglycemia on D-Ribose administration, the D-Glucose may be eliminated. It is suggested that the agent be given one to seven days before and one to seven days after anaesthetic is delivered. Many subjects may have self administered ribose for a longer period. Therefore the method is not limited to the minimal times given, but includes long-term ribose administration both before and after the anaesthetic procedure.

### Example 1. Preparation of substantially pure pyrogen-free ribose. (Reference)

Products produced by fermentation generally have some residue of pyrogens, that is, substances that can induce fever when administered intravenously. Among the most frequent pyrogenic contaminants are bacterial 5 endotoxins. Therefore, endotoxin analysis is used to determine whether a substance is or is not essentially free of pyrogens. Additionally, congeners, that is, undesirable side products produced during fermentation and heavy metals may be carried through and present in the fermentation product.

D-Ribose prepared by fermentation and purified is approximately 97% pure and may contain varying levels of endotoxin. While this product is safe for oral ingestion and may be termed "food grade" it is not "pharma grade," suitable for intravenous administration. D-Ribose may be purified to pharma grade and rendered pyrogen-free. Briefly, all equipment is scrupulously cleaned with a final rinse of pyrogen-free water, which may be double distilled or prepared by reverse osmosis. All solutions and reagents are made up with pyrogen-free water.

A solution of about 30% to 40% ribose in water is prepared. Activated charcoal is added and the suspension mixed at least 30 minutes, while maintaining the temperature at 50-60°C. The charcoal is removed by filtration. The filtered solution should be clear and almost colorless.

Ethanol is added to induce crystallization and the crystals allowed to grow for one or two days. For convenient handling, the crystals are ground and transferred to drums, bags or other containers. Each container is preferably supplied with a bag of desiccant. The final product is essentially pure and free of pyrogens, heavy metals and congeners.

### Example 2. Recovery from anaesthesia

During deep anaesthesia, all bodily functions are depressed. After any prolonged general anaesthesia, that is, anaesthesia where the human patient is unconscious for at least three hours, recovery to full energetic state may require a full month or more. Fpr purposes of describing this invention, by "recovery" is meant the ability of a patient subjected to general anaesthesia to resume normal alertness, ambulatory function and eating. If the patient experiences pain from a surgical procedure, an important aspect of recovery is relief from pain. Hendricks et al (Resuscitation 1984 November: 12(3):213-21, the teachings of which are hereby incorporated by reference) found that rats anesthetized with halothane for 30 minutes showed reduced spontaneous activity and neurological deficit during the first week after anaesthesia. The authors concluded that halothane and nitrous oxide have prolonged effects on locomotor behavior beyond the immediate post-anaesthesia recovery period. Similar effects are frequently observed in human patients after surgery. Patients find that they need more sleep, get fatigued easily throughout a day and are not alert enough to drive an automobile for several weeks. In addition, postoperative pain may require prolonged use of analgesic drugs, which may further inhibit physical activity, as patients tend to be more sedentary to minimize pain. As can be seen in Example 4, not all the effects shown in cardiac surgery wherein the heart is cross-clamped, with resultant decrease in heart function due to ischemia may be due to the ischemia alone. As noted, patients not subjected to ischemia and therefore assumed to have more normal heart function, also benefited from ribose administration as shown by better cardiac outcome. Other aspects of recovery from anaesthesia were not recorded in that trial. Trials were performed to determine whether the better function beyond cardiac parameters due to ribose administration can be shown in other cases of general anaesthesia.

### A. Anecdotal results from non-cardiac surgery with general anaesthesia.

Anecdotal reports have indicated that the administration of D-Ribose hastens recovery to a full energetic state and further, that the degree and duration of pain episodes seem to be lessened. For example, a 69 year-old woman underwent two hip replacement operations, five months apart. With the second operation, she began taking oral ribose immediately after her recovery from anaesthesia. Her recovery to a feeling of alertness and energy was more rapid than after the first operation. Furthermore, her level of pain was less. Likewise, a 52 year-old man also underwent two knee replacement operations. With the second operation, he self-administered D-Ribose pre- and postoperatively. His recovery to a feeling of alertness and energy was more rapid than after the first operation. Bauer et al. (Z. Geb. Neonatal 2001 May-Jun, 205(3):80-85) studied the efficacy of oral glucose for treating procedural pain in neonates. They found that placing a solution of glucose on the tongue of the infant reduced the degree of pain experienced during venous blood sampling. The authors proposed that the orogustatory stimulation by the sweet taste caused an endorphin release. It is not known whether the result seen was due to the local effect or to a systemic effect of glucose.

### B. Cardiac surgery, sheep study.

A study on aortic valve replacement in sheep was carried out. Fourteen cross-bred (male and female) sheep (age range 25 to 68 weeks, body range 47 to 68 kg) were used in these studies. There were two postoperative deaths. The mean CPB time was two hours. (1 Heart Valve Disease Vol 9. No 6, November 2000, the teachings of which are incorporated by reference). The surgical protocol was as follows: Two days before surgery, each animal was given an intramuscular injection of antibiotic: ticarcillin disodium, 0.03 g/kg (SmithKline Beecham Pharmaceuticals, Philadelphia) and Gentocin 1 mg/kg (Fermenta Veterinary Products, Kansas City, MO). On the day of surgery, each animal was given an intramuscular injection of Gentocin 1 mg/kg and atropine sulfate (Medco, St. Joseph, MO), 5 ml of a 2% w/v solution in normal saline. A peripheral intravenous line was inserted. Sodium pentothal (2.5%, Abbott Laboratories, North Chicago, IL) and ticarcillin disodium (0.03 g/kg). General anaesthesia was maintained with isoflurane and supplemental oxygen with further doses of sodium pentothal administered as necessary. The animals were intubated and ventilatory support established. Succinylcholine was given before any incision was made.

The usual intrasurgical parameters were followed, among which were EEG, rectal and esophageal temperatures, serial arterial blood gas. The animal was placed on cardiopulmonary bypass using a Maxima® hollow fiber membrane oxygenator with venous reservoir pump and a BioMedicus 80 constrained vortex centrifugal pump. Cooling was initiated. When adequate cooling had occurred, an aortic cross clamp was applied across the distal ascending aorta and cold (4° C) cardioplegia with 10 meq KCl (Plegisol, Abbott Laboratories) was administered proximal to the applied aortic cross clamp, ice slush was placed over and around the heart, which arrested immediately. The ascending aorta was completely transected transversely, proximal to the cross clamp. During the procedure, further doses of cardioplegia were administered at about 20 to 25 minute intervals directly into each coronary ostia. The aortic leaflets were excised and the annulus of the valve was sized for selection of the prosthetic valve. Prosthetic aortic valves (19 mm) were implanted in each animal, with interrupted, everting, abutting, mattress Ethibond suture being placed into the annulus of the aortic valve and thereafter placed into the skirt of the selected prosthetic valve. The transected aorta was reapproximated and sutured. The circulated blood was rewarmed to 42°C and the heart defibrillated. Once the animal was off CPB and hemodynamically stable, the chest was closed. Ventilation was continued until the animal could breathe spontaneously. When the animal was judged to be alert, the endotracheal tube was removed. The mean time to extubation was about 3 to 4 hours after chest closure. Solid food was provided and the animals observed. The average animal remained quiet and inactive for an additional 2 hours and it was observed that food was not eaten until about 2 ½ to 3 hours after extubation.

In order to determine whether the administration of ribose could shorten the postsurgical recovery time, six cross-bred (male and female) sheep (age range 25 to 68 weeks, body range 46 to 65 kg) were administered pyrogen-free 5% D-Ribose in dextrose 5% water by intravenous infusion at a rate of 100 cc/hour from the time the pre-operative drip was inserted until it was withdrawn. In this series of surgical procedures, the mean time on CPB was slightly longer, from 2 ½ to 3 hours. Nonetheless, the mean time to extubation was reduced to 1 ½ to 2 ½ hours. The animals were monitored with cardiac output, blood pressure, and observation of myocardial relaxation state during and following cardioplegia, time to cardiac arrest with cardioplegia, the time interval between cardioplegic infusions, and the degree of vigorous contractility following defibrillation of the heart at the completion of surgery.

All animals tolerated the supplemental ribose with no metabolic or chemical abnormalities. The infusion of cardioplegia containing ribose resulting a somewhat faster cardiac arrest than in the animals not given ribose, the difference not being statistically significant. The heart was defibrillated easily. The heart was able increase function quickly and to be taken off bypass. Upon weaning from the ventilator, the animals were able to assume a consciousness state faster than the animals not given ribose, were then extubated, The animals were quiet and inactive for only about one hour, increased their activity by standing and even showing ambulaotary activity. Some began eating solid food within the next hour. Due to the increased activity and an interest in eating, it was assumed that the level of pain was less as was reported by the human subjects of Example 5A.

### C. Vascular graft placement.

Adult sheep or canines will be used as an animal model for the effect of ribose on recovery of animals undergoing vascular grafts. The vascular grafts will vary, some being of artificial materials, such as Dacron, and some being of natural blood vessels taken from a donor animal. After passing the animal under general anaesthesia, as in Example 5 B, a neck cutdown will be performed, isolating both the common carotid artery and jugular vein. An arterial catheter will be placed into the common carotid artery for blood pressure monitoring and subsequent blood sampling. A venous catheter will be placed into the jugular vein. Pyrogen-free D-Ribose or D-Glucose (each at 12.5 gm/l) will be administered intravenously at the commencement of the operation at a rate of 100 cc/hour. Both groins of the animal will be shaved, prepped and draped sterilely. Generous left and right groin cutdowns will be performed. Both femoral arteries (left and right) will be isolated and looped with umbilical tapes, both proximally and distally. Distal muscle biopsies will be obtained from both limbs of the animal./ these biopsies will be frozen immediately for adenine nucleotide analysis. The animal will receive acceptable systemic heparinization, as determined by ACT vaslues. A bolus 400 cc injection of pyrogen-free D-Ribose or D-Glucose (each 7 gm/l) will be performed. Vascular clamps will be applied both proximally and distally on each isolated femoral artery. A segment of native artery will be excised and an interposed segment of graft material will be tailored and sewn in place using a running suturing technique. Each anastomosis will incorporate two sutures, each running 180 degrees and tied to each other.

At the completion of each anastomosis, the vascular clamps will be removed in a specific order to make sure that any residual air has been evacuated. Another bolus 400 cc injection of pyrogen-free D-Ribose or D-Glucose (each 7 gm/l) will be given into the proximal femoral artery area. The same test substance will be used in the appropriate limb as determined at the time of the first bolus, prior to the anastomoses. Hemodynamic and fluoroscopic assessments will be made during the healing time to ascertain patency and integrity of the grafts.

The recovery of the animals will be monitored to determine whether the test animals can be extubated sooner, appear alert sooner and move voluntarily. Additional boliAnalgesics will be given for pain as indicated by the behavior of the animals.

### D. Non-cardiac surgery, rat study.

In order to ascertain more definitively whether these results seen in sections B and C above are due to improvement in cardiac function or to improvement in the deficits due to general anaesthesia as indicated in section A above, the following study was designed. Littered-paired Wistar rats will be preconditioned with oral D-Ribose (250 mg/day, 10 animals) as a test drug or D-Glucose(250 mg/day, 10 animals) as a placebo for five days. Following the preconditioning, the rats will be anesthetized with halothane, intubated for artificial respiration and paralyzed with curare. Following general anaesthesia, the rats will be given either the test drug or placebo, intravenously (IV). A two-inch abdominal incision will be made and the viscera will be carefully manipulated to simulate an abdominal exploratory surgery. The incision will be closed and the animals will be held under anaesthesia for one additional hour. Following that hour, anaesthesia will be discontinued and the IV infusion will be halted. The animals will be placed individually in activity cages and their activity will be assessed daily for five days. Test drug or placebo will be added to the drinking water at a dosage of 5% wt/vol. The blinded results will be observed for: first movement (return to consciousness following the sham operation) and daily activity over the first day and next five days. Food and water intake and gastrointestinal function will be measured.

It is expected that the rats given D-Ribose before, during and after the sham operation will demonstrate earlier movement after anaesthesia and increased activity during the following five days, indicating that their recovery level is higher than that of the placebo controls and/or their experienced pain is lessened.

## Claims

1. D-Ribose for administration to patients undergoing general anaesthesia for use in achieving quicker recovery to normal alertness.

2. D-Ribose for use according to claim 1, wherein the ribose is for oral administration before and after general anaesthesia.

3. D-Ribose for use according to claim 2, wherein the ribose is for administration two to four times daily in an amount of 2-10 grams.

4. D-Ribose for use according to claim 1, wherein the ribose is pyrogen-free and is for intravenous administration during and after general anaesthesia.

5. D-Ribose for use according to claim 4, wherein the ribose is pyrogen-free and is for intravenous administration.

6. D-Ribose for use according to claim 5, wherein the ribose is in a composition which further comprises D-glucose.

7. D-Ribose for use according to claim 6, wherein the composition comprises 5-10% pyrogen-free D-ribose and 5-10% D-glucose.

8. D-Ribose for use according to claim 1, wherein the D-ribose is to be administered orally while the mammal is able to ingest D-ribose, and pyrogen-free D-ribose is to be administered intravenously while the mammal is unconscious or otherwise unable to ingest D-ribose.

9. D-Ribose for use according to claim 8, wherein the ribose is for oral administration two to four times daily in an amount of 2-10 grams or for intravenous administration in an amount of 20-300 mg/kg/hour.

## Patentansprüche

1. D-Ribose für die Verabreichung an Patienten, die einer Allgemeinanästhesie ausgesetzt sind, für die Verwendung zum Erreichen einer schnelleren Erholung hin zu normaler Aufmerksamkeit.

2. D-Ribose für die Verwendung nach Anspruch 1, wobei die Ribose zur oralen Verabreichung vor und nach der Allgemeinanästhesie ist.

3. D-Ribose für die Verwendung nach Anspruch 2, wobei die Ribose zur Verabreichung zwei- bis viermal täglich in einer Menge von 2-10 Gramm ist.

4. D-Ribose für die Verwendung nach Anspruch 1, wobei die Ribose pyrogenfrei und zur intravenösen Verabreichung während und nach der Allgemeinanästhesie ist.

5. D-Ribose für die Verwendung nach Anspruch 4, wobei die Ribose pyrogenfrei und für die intravenöse Verabreichung ist.

6. D-Ribose für die Verwendung nach Anspruch 5, wobei die Ribose in einer Zusammensetzung ist, die ferner D-Glukose umfasst.

7. D-Ribose für die Verwendung nach Anspruch 6, wobei die Zusammensetzung 5-10% pyrogenfreie D-Ribose und 5-10% D-Glukose umfasst.

8. D-Ribose für die Verwendung nach Anspruch 1, wobei die D-Ribose oral verabreicht wird, solange das Säugetier in der Lage ist die D-Ribose einzunehmen, und die pyrogenfreie D-Ribose intravenös verabreicht wird, solange das Säugetier nicht bei Bewusstsein ist oder aus irgendeinem anderen Grund nicht in der Lage ist die D-Ribose einzunehmen.

9. D-Ribose für die Verwendung nach Anspruch 8, wobei die Ribose zur oralen Verabreichung zwei- bis viermal täglich in einer Menge von 2-10 Gramm gegeben wird oder zur intravenösen Verabreichung in einer Menge von 20-300 mg/kg/h gegeben wird.

## Revendications

1. D-ribose destiné à une administration à des patients subissant une anesthésie générale destiné à être utilisé pour parvenir à récupérer plus rapidement une vigilance normale.

2. D-ribose destiné à être utilisé selon la revendication 1, dans lequel le ribose est destiné à une administration orale avant et après l'anesthésie générale.

3. D-ribose destiné à être utilisé selon la revendication 2, dans lequel le ribose est destiné à une administration deux à quatre fois par jour en une quantité de 2 à 10 grammes.

4. D-ribose destiné à être utilisé selon la revendication 1, dans lequel le ribose est sans pyrogène et est destiné à une administration par voie intraveineuse pendant et après l'anesthésie générale.

5. D-ribose destiné à être utilisé selon la revendication 4, dans lequel le ribose est sans pyrogène et est destiné à une administration intraveineuse.

6. D-ribose destiné à être utilisé selon la revendication 5, dans lequel le ribose est dans une composition qui comprend en outre du D-glucose.

7. D-ribose destiné à être utilisé selon la revendication 6, dans lequel la composition comprend 5 à 10 % de D-ribose sans pyrogène et 5 à 10 % de D-glucose.

8. D-ribose destiné à être utilisé selon la revendication 1, dans lequel le D-ribose doit être administré par voie orale lorsque le mammifère est capable d'ingérer du D-ribose, et le D-ribose sans pyrogène doit être administré par voie intraveineuse lorsque le mammifère est inconscient ou sinon n'est pas capable d'ingérer le D-ribose.

9. D-ribose destiné à être utilisé selon la revendication 8, dans lequel le ribose est destiné à une administration orale deux à quatre fois par jour dans une quantité de 2 à 10 grammes ou destiné à une administration intraveineuse en une quantité de 20 à 300 mg/kg/heure.
